# EUROPÄISCHE PATENTSCHRIFT

(11) **EP 2 497 427 B1**
(45) Veröffentlichungstag und Bekanntmachung des Hinweises auf die Patenterteilung: **15.01.2020**
(21) Anmeldenummer: 11157710.2
(22) Anmeldetag: 10.03.2011
(51) Int. Cl.: A61B 17/00, A61B 17/3203, A61B 18/00, A61B 18/02, A61B 18/14

(54) **Chirurgisches Instrument mit digitaler Datenschnittstelle**
Surgical instrument with digital data interface
Instrument chirurgical doté d'une interface numérique

(43) Veröffentlichungstag der Anmeldung: 12.09.2012
(73) Patentinhaber: Erbe Elektromedizin GmbH, 72072 Tübingen (DE)
(72) Erfinder: Baur, Thomas, 70128, Rottenburg-Hailfingen (DE); Kegreiss, Marc, 72072, Tübingen (DE); Selig, Peter, 72379, Hechingen (DE)
(74) Vertreter: Rüger Abel Patentanwälte PartGmbB

(56) Entgegenhaltungen:
- WO-A1-2008/131357
- WO-A2-2008/098085
- US-A1- 2003 069 571
- US-A1- 2008 077 158

## Beschreibung

Die Erfindung betrifft ein chirurgisches Instrument sowie das aus dem chirurgischen Instrument und dem speisenden Chirurgiegerät bestehende System.

In der Chirurgie werden verschiedene Instrumente eingesetzt, die innerhalb eines chirurgischen Systems betrieben und dabei von einem Chirurgiegerät gespeist werden. Solche chirurgischen Instrumente sind z.B. mit Elektroden versehene Instrumente für die HF-Chirurgie oder auch Applikatoren, mit denen anstelle von elektrischem Strom einem biologischen Gewebe andere Medien an einer Wirkstelle des Operationsgebiets zugeführt werden. Solche Wirkmedien können z.B. Wasser, Kältemedien oder ähnliches sein. Zwischen dem speisenden Chirurgiegerät und dem jeweils eingesetzten Instrument ist eine Leitung für den elektrischen Strom oder das sonstige Wirkmedium vorhanden.

Zum Betrieb an einem Chirurgiegerät sind meist verschiedene Instrumente und Zubehörteile vorgesehen, die an diesem Gerät betrieben werden können. Die Instrumente können unterschiedlicher Natur sein. Es sind bspw. sogenannte monopolare Handgriffe in Gebrauch, die als elektrisches Skalpell, als Koagulationsinstrument, als Resektionsinstrument oder ähnliches ausgebildet sein können. Dazu werden häufig auch auswechselbare Elektroden vorgesehen. Es sind auch bipolare Instrumente, wie z.B. Pinzetten, Fasszangen und dergleichen zur Koagulation von Blutgefäßen, zur Durchtrennung derselben oder zu ähnlichen Zwecken in Gebrauch. Die verschiedenen Instrumente benötigen in der Regel jeweils individuelle spezifische Betriebsarten zur elektrischen Ansteuerung.

Die WO 2008/131357 offenbart eine Operationseinheit, die verschiedene Operationsinstrumente wie Irrigations-, Beleuchtungs- und Schneidwerkzeuge bereitstellt, mit diesen in Verbindung steht und diese wenn nötig z.B. mit Spannung versorgt, Daten austauscht oder Medien zu den Operationsinstrumenten hinführt (z.B. bei einer Spülung) oder von den Operationsinstrumenten wegführt (z.B. bei einer Absaugung). Dazu werden kabellose Übertragung (z.B. 801.11 oder Bluetooth) oder drahtgebundene Übertragung mit z.B. seriellem Bus oder parallelem Bus vorgeschlagen. Es wird eine Ausführungsform als vorteilhaft offenbart, in der die in der Operationseinheit interne Leistungsquelle von einer Batterie gebildet und diese auch zur Versorgung der Operationsinstrumente verwendet wird. Hintergrund der Offenbarung ist ein chirurgisches Verfahren am Auge mit einem Vitrektom, dass oftmals pneumatisch betrieben wird, das aber auch e-lektro-magnetisch ausgeführt sein kann.

Weiterhin wird in der US 2008/0077158 A1 ein Verfahren und eine Vorrichtung zur computergestützten Chirurgie beschrieben. Das chirurgische Instrument weist eine drahtlose Verbindung, z.B. eine Bluetooth-Verbindung, zu einem Computer auf, der dem Chirurgen assistieren oder ein oder mehrere chirurgische Instrumente kontrollieren kann.

Mit dem chirurgischen Kontrollsystem aus der WO 2008/098085 A2 kann der Chirurg ein einziges Fußpedal zur Kontrolle eines einzelnen chirurgischen Instruments aus einer Vielzahl von erforderlichen Chirurgieinstrumenten verwenden. Bei Bedarf kann der Chirurg eine Verbindung von dem Fußpedal zu einem bestimmten chirurgischen Instrument herstellen und das Instrument mit dem Pedal bedienen.

Druckschrift US 2003/0069571 A1 offenbart ein leichtgewichtiges und daher tragbares chirurgisches Instrument und ein Verfahren zum Versiegeln und Verbinden oder zum Trennen von Gewebe unter Druck- und Hitzeanwendung bevorzugt mit einer Spannungsquelle mit handelsüblichen Batterien (z.B. AA-Zellen). Eine Leitung überträgt Leistung von der Spannungsquelle zum chirurgischen Instrument.

Es wird angestrebt sicherzustellen, dass das Instrument nicht in ungeeignetem Mode betrieben wird. Deshalb schlägt die WO 2009/074329 A2 vor, dem Instrument einen RFID-Transponder zuzuordnen, der mit einer Instrumentenantenne in Verbindung steht. In dem chirurgischen Gerät ist eine Sende-/Empfangseinheit angeordnet, die mit dem RFID-Transponder kommuniziert und auf diese Weise das angeschlossene Instrument erkennt. Weiter schlägt diese Druckschrift vor, über den RFID-Transponder Befehle von Schaltern an das Chirurgiegerät zu übertragen, wobei die Schalter an dem Instrument angeordnet sind. Auch kann das Instrument Sensoren enthalten, deren Signale aufbereitet und über den RFID-Transponder an das Chirurgiegerät gegeben werden. Der RFID-Transponder wird dabei möglichst gerätenah angeordnet, vorzugsweise in dem an das Chirurgiegerät anzuschließenden Stecker oder in dem Verbindungskabel.

Weiter ist aus der US 7,503,917 B2 ein chirurgisches Instrument bekannt, das mehrere Bedienschalter enthält. Diese sind über Signalleitungen mit dem Chirurgiegerät verbunden. Die Bedienschalter schließen einzelne Widerstände einer Spannungsteilerkaskade kurz und geben somit an den Signalleitungen ein charakteristisches Spannungssignal ab, aus dem das Chirurgiegerät die Schalterbetätigung erkennt und bestimmten Schaltern zuordnet.

Die elektrische Umgebung ist in einem Operationssaal, zumindest zeitweilig, sehr stark gestört. Hochfrequente Spannungen und Ströme führen in unmittelbarer Umgebung des Chirurgiegeräts und des chirurgischen Instruments sowie des Verbindungskabels zu hohen Störfeldstärken, die die Signalübertragung negativ beeinflussen können.

Auch die US 7,479,140 B2 nutzt zur Übertragung von Schalterbefehlen von dem chirurgischen Instrument an das Gerät Analogsignalleitungen, über die verschiedene charakteristische Widerstände mit dem Chirurgiegerät verbunden werden.

Aus der DE 10 2005 044 918 A1 ist außerdem ein System zur kontaktlosen Identifikation und Kommunikation zwischen einem Chirurgiegerät und einem angeschlossenen Instrument bekannt. Wiederum wird ein Transponder eingesetzt, der in dem Instrumentenstecker des chirurgischen Instruments angeordnet ist. In der zugeordneten Gerätebuchse ist die mit dem Transponder kommunizierende Leseeinheit vorgesehen. Viele Einstellungen des Chirurgiegeräts müssen unmittelbar an demselben vorgenommen werden. Der Operateur hat an dem Instrument nur wenige Einstellmöglichkeiten.

Davon ausgehend ist es Aufgabe der Erfindung, ein chirurgisches Instrument zu schaffen, das verbesserte Einstellmöglichkeiten zulässt.

Weiter soll ein Chirurgiegerät angegeben werden, das mit dem Instrument zusammenwirkt.

Schließlich soll ein Chirurgiesystem geschaffen werden, das aus einem Chirurgiegerät und mindestens einem chirurgischen Instrument mit komfortablen Einstellmöglichkeiten an dem Instrument besteht.

Die Lösung dieser Aufgaben wird durch die Schaffung eines chirurgischen Instruments ermöglicht mit den Merkmalen entsprechend Anspruch 1 und weiteren besonderen Ausführungsformen entsprechend den Unteransprüchen. Das Instrument enthält mindestens einen Mikrocontroller, der in dem Handgriff des chirurgischen Instruments angeordnet ist und der ohne Zwischenschaltung einer Funkstrecke über die ohnehin zwischen dem Chirurgiegerät und dem chirurgischen Instrument vorhandene Leitung mit dem Chirurgiegerät kommuniziert.

Anstelle eines Mikrocontrollers werden auch andere programmierbare Bausteine wie beispielweise DSP, FPGA, CPLD, ASIC verstanden.

Die Unterbringung des Mikrocontrollers in dem Handgriff ermöglicht es, an dem Handgriff Bedienelemente vorzusehen, mit denen sich viele Funktionen des Chirurgiegeräts steuern lassen. Beispielsweise können Schalter vorgesehen werden, deren Betätigung situationsabhängig konfigurierbar ist. So kann jeder Schalter mit mehreren Funktionen belegt werden, wobei die Auswahl, welche der Funktionen durch Schalterbetätigung aktiviert wird, zum Beispiel von der Betriebsart, der konkreten Situation, der Betätigung anderer Schalter oder dergleichen abhängt. Beispielsweise kann ein chirurgisches Instrument einen Beschleunigungssensor enthalten, der die räumliche Position des chirurgischen Instruments, bspw. ob es vertikal oder horizontal gehalten wird, erkennt und davon abhängig Funktionen steuert. Zum Beispiel kann auf diese Weise eine von mehreren Funktionen eines Tasters ausgewählt werden. Der Mikrocontroller kann außerdem dazu genutzt werden, ein Identifikationssignal zu generieren und an das Chirurgiegerät zu senden, um diesem mitzuteilen, welches Instrument bzw. welcher Instrumententyp, welches Funktionselement, welche Elektrode usw., an das Chirurgiegerät angeschlossen ist. Eine entsprechende mit dem Mikrocontroller kommunizierende Identifikationseinrichtung kann z.B. an einer Wechselkupplung des Handgriffs vorgesehen sein, an die sich verschiedene Funktionselemente wie beispielsweise Elektroden anschließen lassen. Der Mikrocontroller kann mittels der Identifikationseinrichtung das angeschlossene Funktionselement erkennen und entsprechende Befehle, Signale oder dergleichen an das Chirurgiegerät senden, so dass dieses gegebenenfalls automatisch entsprechende Einstellungen vornehmen kann.

Weiter kann der Mikrocontroller mit einer Anzeigeeinrichtung versehen sein, die unmittelbar an dem Handgriff verschiedene Anzeigen erbringt. Es kann sich dabei um einzelne Kontrollleuchten, Displays oder dergleichen, handeln. Die Anzeigeeinrichtung kann außer oder anstelle von optischen Anzeigemitteln auch sensorische oder akustische Anzeigemittel umfassen. Solche können bspw. Summer, Piezo-Schallwandler oder Vibrationsgeber sein, um dem Operateur akustische oder an dem Handgriff taktil fühlbare Signale zu geben. Die Anzeigeeinrichtung kann auch mit der Bedieneinrichtung kombiniert sein. Z.B. können beleuchtete Tasten vorgesehen sein, die während oder nach einer Betätigung oder abhängig von anderen Ereignissen, Messwerten, Zuständen aufleuchten, verlöschen, ihre Leuchtfarbe ändern oder dergleichen.

Die von dem Chirurgiegerät zu dem chirurgischen Instrument führende Leitung kann eine Licht oder Fluid (flüssig oder gasförmig) führende Leitung oder dergleichen sein. Im bevorzugten Fall handelt es sich jedoch um eine elektrische Leitung mit mindestens einer Ader, die eine zur Erzielung eines Gewebeeffektes geeignete Spannung bzw. einen geeigneten Strom, führt. Im bevorzugten Fall ist dies eine HF-Spannung bzw. ein HF-Strom ausreichender Leistungsfähigkeit. Die Frequenz liegt üblicherweise zwischen 100 kHz und 5 MHz. Die Spannung kann mehrere 1000 V betragen.

Die Leitung enthält ein Paar Adern zur Datenübertragung und vorzugsweise mindestens eine weitere Ader zur Spannungsversorgung des Mikrocontrollers. Vorzugsweise werden die Daten auf den beiden Leitungen in Form von Strömen übertragen. Die Ströme oder Signale auf zwei datenübertragenden Leitungen, die ein Paar bilden, haben zueinander entgegengesetzte Richtungen bzw. sind physikalisch zueinander invertiert. Dies ermöglicht eine sichere Datenübertragung mit hoher Datenübertragungsrate auch in intensiv gestörter Umgebung, insbesondere in unmittelbarer Nachbarschaft zu der den hochfrequenten Strom führenden Ader der gleichen Leitung. Die Leitung kann einige Meter lang sein, so dass es hier auf besondere Störfestigkeit ankommt. Mit der vorgestellten Maßnahme kann eine sichere Datenübertragung ohne komplizierte Abschirmungen oder dergleichen Maßnahmen erreicht werden.

Mit den beiden Leitungen werden die Daten von dem Mikrocontroller zu dem Chirurgiegerät und umgekehrt vorzugsweise als serieller Datenstrom übertragen. Es ist möglich, die Datenübertragung sowohl bei nicht aktivierter Elektrode des chirurgischen Instruments wie auch bei aktivierter Elektrode zu übertragen.

Die Anordnung einer Anzeigeeinrichtung an dem chirurgischen Instrument ermöglicht die Datenübertragung von dem Chirurgiegerät an das Instrument, um dort Informationen wiederzugeben, die bislang lediglich an einem Bildschirm oder an einer sonstigen Anzeigeeinrichtung des Chirurgiegeräts selbst wiedergegeben worden sind. Dies ermöglicht dem Operateur eine sehr weit reichende Bedienung des Chirurgiegeräts ohne den Blick aus seinem Operationsfeld abzuwenden oder die Einstellung per Befehl durch Dritte vornehmen zu lassen.

Ein erfindungsgemäßes HF-Chirurgie-Instrument kann in der Form ausgestaltet sein, dass es für monopolare und/oder bipolare Anwendungen oder eine Kombination dieser beiden verwendet werden kann.

Weitere Einzelheiten vorteilhafter Ausführungsformen der Erfindung ergeben sich aus der Zeichnung der Beschreibung oder Unteransprüchen. Es zeigen:
Figur 1 ein chirurgisches System, bestehend aus Chirurgiegerät und chirurgischem Instrument, verbunden über eine Leitung, in schematischer Blockdarstellung,
Figur 2 eine Eingabeeinrichtung des chirurgischen Instruments nach Figur 1, in schematisierter Darstellung,
Figur 3 die Leitung zur Verbindung des chirurgischen Instruments mit dem Chirurgiegerät, in schematisierter Querschnittsdarstellung,
Figur 4 ein Strom-Signaldiagramm zur Veranschaulichung des Datenflusses zwischen dem chirurgischen Instrument und dem Chirurgiegerät und
Figur 5 ein Spannungs-Signaldiagramm zur Veranschaulichung des Datenflusses zwischen dem chirurgischen Instrument und dem Chirurgiegerät.

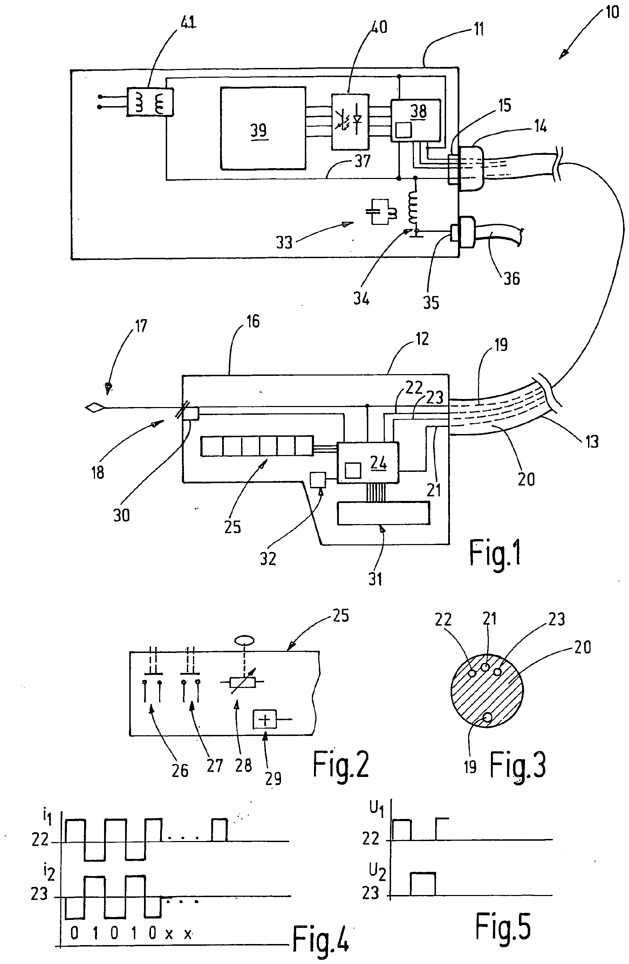

In Figur 1 ist ein chirurgisches System 10 mit einem Chirurgiegerät 11 und einem chirurgischen Instrument 12 schematisch veranschaulicht. Zur Verdeutlichung wird die Erfindung hier anhand eines HF-chirurgischen Systems erläutert, bei dem eine hochfrequente Hochspannung das an dem chirurgischen Instrument wirksame Wirkmedium bildet. Die Erfindung ist darauf nicht beschränkt. Die nachfolgend erläuterten Einzelheiten können auch an einem Instrument mit anderem Wirkmedium, insbesondere unter Druck stehendem Wasser, Kryofluid oder dergleichen, vorgesehen werden.

Das chirurgische Instrument 12 und das Chirurgiegerät 11 sind untereinander durch eine Leitung 13 verbunden, deren Länge so bemessen ist, dass das Chirurgiegerät 11 in einigem Abstand von dem Patienten beispielweise im nicht sterilen Bereich aufgestellt werden kann, während mit dem chirurgischen Instrument 12 am Patienten gearbeitet wird. Die Leitung 13 ist über einen Stecker 14 mit einer Buchse 15 des Chirurgiegeräts 11 verbunden. An die Buchse 15 können auf diese Weise verschiedene chirurgische Instrumente angeschlossen werden, um verschiedene Operationen vorzunehmen.

Im vorliegenden Ausführungs- und Erläuterungsbeispiel ist das chirurgische Instrument 12 als monopolares Instrument ausgebildet. Es weist einen Handgriff 16 auf, an dem ein Werkzeug in Form einer Elektrode 17 gehalten ist. Die Elektrode 17 kann bspw. eine Schneideelektrode in Form eines Spatels oder einer Nadel, wie bspw. eine Koagulationselektrode in Form einer Kugel oder dergleichen, sein. Zur Halterung der Elektrode 17 an dem Handgriff 16 kann eine Kupplung 18 vorgesehen sein, die in Figur 1 lediglich symbolisch angedeutet ist.

Vorzugsweise von der Kupplung 18 gegenüber liegenden Seite des Handgriffs 16 erstreckt sich die Leitung bzw. das Kabel 13 weg. Es enthält mindestens einen Leitungskanal zur Zuleitung der zur Applikation an dem biologischen Gewebe vorgesehenen Energieform von dem Chirurgiegerät 11 zu dem chirurgischen Instrument 12. Dieser Leitungskanal ist im vorliegenden Ausführungsbeispiel als Ader 19, d.h. als elektrischer, von Isolierstoff 20 (Figur 3) umgebener Leiter ausgebildet. Nachdem das Instrument 12 hier als HFchirurgisches Instrument veranschaulicht ist, führt der Leiter 19, zumindest zeitweilig hochfrequente Hochspannung. Unter "Hochspannung" wird hierbei jede Spannung oberhalb einer Schutzkleinspannung von 42 V verstanden. In vielen Fällen liegt der Spitzenwert dieser Hochspannung im Bereich von mehreren 1000 V.

Die Leitung 13 enthält weitere Adern 21, 22, 23, die als in den Isolierstoff 20 eingebettete elektrische Leiter ausgebildet sind.

In dem Handgriff 12 ist ein Mikrocontroller 24 angeordnet. Dieser kann einen Speicher enthalten oder mit einem gesonderten Speicher (EPROM) kommunizieren. Der Mikrocontroller 24 ist vorzugsweise mit seinem Masseanschluss an die Ader 19, mit zwei Ein- und Ausgabeports an die Adern 22, 23 und mit seinem Betriebsspannungsanschluss an die Ader 21 angeschlossen, welche die erforderliche elektrische Energie zum Betrieb des Mikrocontrollers führt.

Der Mikrocontroller 24 ist vorzugsweise permanent in Betrieb, sobald das Instrument 12 an das Chirurgiegerät 11 angeschlossen und das Chirurgiegerät 11 eingeschaltet ist. Der Mikrocontroller 24 kann mit einem Programm versehen sein, das entweder beim Einschalten einmalig oder alternativ auch von Zeit zu Zeit ein Identifizierungssignal an das Chirurgiegerät 11 sendet, um sich zu identifizieren und damit dem Chirurgiegerät 11 Information über den Typ des angeschlossenen Instruments zu geben. Das Identifizierungssignal wird als Identifizierungscode gesendet. In dem Chirurgiegerät 11 kann der Identifizierungscode ausgewertet werden, um eine zu dem Instrument 12 passende Voreinstellung und/oder Vorauswahl passender Betriebsarten, Moden, Effekte usw. vorzunehmen.

An dem Handgriff 16 können Bedienelemente 25 vorgesehen sein, zu denen eine oder mehrere Tasten, ein oder mehrere Drehknöpfe, ein oder mehrere Lagesensoren, ein oder mehrere Beschleunigungssensoren oder dergleichen, oder eine beliebige Kombination der genannten Elemente gehören können. Tasten, Drehknöpfe oder dergleichen können beleuchtet sein oder mit sonstigen Anzeigemitteln kombiniert sein, die an den Mikrocontroller 24 angeschlossen sind. Zusätzlich zu den Bedienelementen 25 können Sensorelemente vorgesehen sein, die bestimmte Messwerte, z.B. Temperaturen erfassen und als Signal an den Mikrocontroller geben. Dieser kann die Messwerte verarbeiten und/oder an das Chirurgiegerät 11 melden.

Figur 2 veranschaulicht solche Bedienungselemente 25 schematisch. Lediglich beispielhaft sind zwei Taster 26, 27, ein Potentiometer 28 und ein Lagesensor 29 veranschaulicht, der z.B. eine horizontale und eine vertikale Positionierung des Handgriffs 16 unterscheiden kann. Die Bedienelemente 25 sind vorzugsweise ausschließlich mit dem Mikrocontroller 24 verbunden. Eine direkte Leitungsverbindung zwischen den Bedienelementen 25 und dem Chirurgiegerät 11 besteht somit nicht.

An der Kupplung 18 kann eine Identifizierungseinrichtung 30 vorgesehen sein, die dazu dient, die Art und/oder Größe des Werkzeuges, im beschriebenen Ausführungsbeispiel, der Elektrode 17 zu erfassen, die an die Kupplung 18 angeschlossen ist. Beispielsweise kann die Identifizierungseinrichtung 30 eine Buchse sein, der ein Kodierstecker der Elektrode 17 zugeordnet ist. Als Identifizierungseinrichtung 30 ist jedes technische Mittel geeignet, das ein den Typ und/oder die Größe des Werkzeuges 17 kodierendes Merkmal erfassen kann. Ein solches Merkmal kann in der Dimension eines entsprechenden Elements, wie z.B. eines Fingers oder Vorsprungs, wie auch in der Anordnung mehrerer Strukturen, wie bspw. Ausnehmungen oder Vorsprünge, liegen.

Der Mikrocontroller 24 kann außerdem mit einer Display- bzw. einer Anzeigeeinrichtung 31 verbunden sein, die bspw. ein LCD-Display, ein LED-Display, eine sonstige graphische Wiedergabeeinrichtung, Kontrolllampen oder dergleichen umfassen kann. Die Displayeinrichtung 31 kann ganz oder teilweise mit den Bedienungselementen 25 kombiniert werden. Beispielweise kann die Displayeinrichtung 31 berührungs- oder druckempfindliche Bereiche aufweisen, um Eingaben zu ermöglichen.

Zur weiteren Signalausgabe können akustische Geber oder auch ein Vibrationsgeber 32 vorgesehen sein, der Vibrationen in den Handgriff 16 einleiten kann. Ein Vibrationsgeber 32 kann bspw. durch einen kleinen mit einem exzentrisch gelagerten Gewicht versehenen Motor gebildet sein.

Das Chirurgiegerät 11 weist einen in Figur 1 lediglich schematisch veranschaulichten Generator 33 auf, der dazu eingerichtet ist, Hochfrequenzleistung abzugeben und über die Buchse 15 in die Ader 19 einzuspeisen. Der Generator 33 liefert seine Hochfrequenzleistung mit Bezug auf Nullpotential 34, das an einer Neutralbuchse 35 liegt. An diese ist eine Leitung 36 angeschlossen, an deren Ende eine nicht weiter veranschaulichte neutrale Elektrode zur großflächigen Befestigung an dem Patienten vorgesehen ist. Die Neutralbuchse 35 kann außerdem zum Anschluss bipolarer Geräte genutzt werden.

Die von dem Generator 33 gespeiste hochfrequenzspannungsführende Leitung 37 ist das Bezugspotential für einen zweiten Mikrocontroller 38, der zur Kommunikation mit dem instrumentenseitigen Mikrocontroller 24 dient. Der Mikrocontroller 38 erhält Betriebsspannung von einer gesonderten Versorgungseinheit 41, z.B. in Gestalt eines Netzteils, die über die Ader 21 auch den instrumentenseitigen Mikrocontroller 24 speist. Über die Buchse 15 ist der Mikrocontroller 38 außerdem an die beiden zur Datenleitung dienenden Adern 22, 23 angeschlossen. Mit der weiteren Gerätesteuerung 39 kommuniziert der Mikrocontroller 38 über eine Potentialtrenneinrichtung 40 bspw. in Gestalt von Optokopplern.

Das insoweit beschriebene Chirurgiesystem 10 arbeitet wie folgt:
Soll ein bestimmtes chirurgisches Instrument 12 benutzt werden, wird dieses durch Einstecken des Steckers 14 an das Chirurgiegerät 11 angeschlossen. Der Mikrocontroller 24 erhält dadurch Betriebsspannung. Er kann nun verschiedene Aktionen ausführen, bspw. einen Identifizierungscode senden. Dabei nutzt er die beiden Adern 22, 23 der Leitung 13 zur Datenkommunikation. Die Pegel auf den beiden Leitungen oder Adern 22, 23 sind in Figur 4 gesondert dargestellt. Die zu übertragenden Bits werden vorzugsweise als Strom- oder Spannungssignale mit gegensinniger Polarität übertragen. Beispielsweise wird ein Null-Bit mit positivem Strom i1 auf der Ader 22 und negativem Strom i2 auf der A-der 23 übertragen. Zur Übertragung einer logischen 1 kehren beide Ströme ihre Polarität um. Führen die beiden Adern 22, 23 keine Ströme, wird kein Bit übertragen. Führt nur eine der beiden Adern 22 Strom, die andere jedoch nicht, wird dies als Störimpuls interpretiert und somit kein gültiges Bit erkannt. Figur 5 veranschaulicht die zugehörigen Spannungen an den Adern 22, 23. Ist die normierte Spannung an der Ader 22 gleich Eins, liegt an der anderen Ader die normierte Spannung von Null an und umgekehrt.

Unabhängig von elektromagnetischen Einstrahlungen kann somit die Datenübertragung schon auf elektronisch-physikalischer Ebene relativ sicher gestaltet werden. Noch erhöht werden kann die Sicherheit, wenn die voneinander isolierten Adern 22, 23 miteinander und gegebenenfalls auch mit der Ader 21 verdrillt sind.

Ein vorhandener Bereitschaftszustand des Instruments 12 kann nun bspw. mittels der Displayeinrichtung 31 an dem Handgriff 16 signalisiert werden. Außerdem kann das Instrument 12 Eingaben entgegennehmen, die sofort oder auch später als Datenstrom an das Chirurgiegerät 11 übertragen werden. Diese Eingaben können bspw. die Wahl verschiedener Moden, die Einstellung der Stärke eines Effekts, die Einstellung bestimmter Betriebsdauern oder Zeiten oder ähnliches sein. Dabei können Mehrfachbelegungen der Elemente 25 vorhanden sein, wobei die Belegungsebenen z.B. durch Auswahltasten, Auswahlschalter oder auch durch Positionierung des Handgriffs 16 in z.B. vertikaler oder horizontaler Lage ausgewählt werden. Letzteres kann der Mikrocontroller 24 über den Lagesensor 29 erfassen. Sind Tasten beleuchtet, können sie abhängig von der Wahl der Belegungsebene ihre Leuchtfarbe wechseln. Die Auswahl der Belegungsebene kann auch anderweitig angezeigt oder quittiert werden.

Die Taster 26, 27 können bspw. dazu genutzt werden, den Generator 33 zu aktivieren und somit Hochfrequenzleistung an der Elektrode 17 bereitzustellen. Wird der Taster 26 dazu genutzt und betätigt, setzt der Mikrocontroller 24 diesen Befehl in einen entsprechenden Datenstrom um, der diesen Befehl kodiert. Der Mikrocontroller 38 empfängt diesen Datenstrom und gibt den Befehl an die Gerätesteuerung 39 weiter. Diese aktiviert den Generator 33 je nach gewählter Betriebsart mit den entsprechenden Spannungs-, Strom- und Leistungsparametern. Wird der Taster 26 losgelassen, erfasst der Mikrocontroller 24 dies und sendet somit einen Stoppbefehl an den Mikrocontroller 38 und somit über diesen an die Gerätesteuerung (39). Entsprechend wird der Generator 33 wieder stillgesetzt. Verstellungen der Art oder Größe des Effekts können ebenfalls durch Eingaben an den Bedienungselementen 25 vorgenommen werden. Vorzugsweise sind die Einstellmöglichkeiten dabei auf die mit dem speziellen Instrument sinnvoll nutzbaren Einstellungen beschränkt. Die Beschränkung kann sowohl von dem Mikrocontroller 24 oder, nach Identifizierung des Instruments 12, auch von dem Chirurgiegerät 11 bewirkt werden.

Die Einstellungen werden über den Mikrocontroller 24 nicht nur an das Chirurgiegerät 11 gemeldet, sondern können, zumindest optional, auch an der Displayeinrichtung 31 wiedergegeben werden. Auf diese Weise kann der Chirurg eine Vielzahl von Einstellungen, die ansonsten nur an dem Chirurgiegerät 11 selbst vorzunehmen waren, nun auch direkt über das chirurgische Instrument 12 vornehmen.

Die Erfindung ermöglicht es, Schaltzustände von Aktivierungselementen, wie bspw. den Bedienelementen 25 oder Messwerte von Sensoren direkt in dem chirurgischen Instrument 12 zu erfassen und aufzubereiten, und dann als digitalen Datenstrom über eine serielle bidirektionale Schnittstelle von dem chirurgischen Instrument 12 zu dem Chirurgiegerät 11 zu schicken. Alternativ können auch Daten von dem Chirurgiegerät 11 zu dem Instrument 12 geschickt werden, um bspw. ein Speicherelement zu beschreiben oder einen Aktor zu betätigen. Es kann dadurch prinzipiell eine beliebige Anzahl von Aktivierungselementen bzw. sonstigen Bedienungselementen 25 vorgesehen werden, ohne die Anzahl der elektrischen Verbindungen zwischen dem chirurgischen Instrument 12 und dem Chirurgiegerät 11 ändern zu müssen. Störempfindliche Analogsignale von Sensoren oder ähnlichen Quellen können direkt in dem chirurgischen Instrument 12 ausgewertet werden. Durch eine vorzugsweise bidirektionale Datenkommunikation können nicht nur Werte von dem chirurgischen Instrument 12 zu dem Chirurgiegerät 11 gemeldet werden, sondern auch Funktionen des Instruments 12 durch das Chirurgiegerät 11 aktiviert werden. Das chirurgische Instrument 12 kann in einem elektronischen Speicher Daten zur Identifikation des jeweiligen Instruments durch das Chirurgiegerät 11 beinhalten.

Letztendlich kommuniziert über eine drahtgebundene, bidirektionale Datenübertragungsstrecke eine elektronische Baugruppe des Chirurgiegeräts 11 mit einer elektronischen Baugruppe in dem chirurgischen Instrument 12. Beide Baugruppen enthalten zumindest vorzugsweise einen Mikrocontroller.

In der stark störungsbehafteten Umgebung eines aktiven HF-Chirurgiegeräts 11, aber auch bei Wasserstrahl- und Kryochirurgiegeräten wird so eine sichere Datenübertragung sichergestellt. Vorzugsweise wird eine differentielle Übertragungstechnik gewählt, bei dem ein oder mehrere Leitungspaare (Adern 22, 23) genutzt werden, von denen jeweils einer der Leiter das Signal und der andere das invertierte Signal überträgt. Durch Subtraktion der beiden Signale wird empfängerseitig das eigentliche Datensignal gewonnen. Koppeln Störungen aus der Umgebung auf die Datenübertragungsstrecke, d.h. die Leitung ein, geschieht dies wegen der räumlichen Nähe und gegebenenfalls Verdrillung der beiden Leitungen im gleichen Maße. Durch die an den jeweiligen Empfängern vorgenommene Subtraktion heben sich die Störungen im Signal auf.

Es kann entweder für jede Richtung der Datenübertragung ein eigenes Leitungspaar oder, wie vorstehend beschrieben, ein einziges Leitungspaar 22, 23 für beide Übertragungsrichtungen vorgesehen werden. Somit ist sowohl Vollduplex- als auch Halbduplexbetrieb möglich.

Es ist möglich die HF-spannungsführende Leitung als Bezugspotential für die Datenübertragung zu nutzen. Das Bezugspotential kann über eine geeignete eigene Ader geführt werden, die geräteseitig, instrumentenseitig oder auch an beiden Seiten mit der HF-Leitung verbunden ist. Es kann alternativ auch die HF-Leitung selbst als Datensignal-Masseleitung gewählt werden.

In der stark störungsbehafteten Umgebung eines aktiven HF-Chirurgiegeräts 11, aber auch bei Wasserstrahl-, Argonplasma- und Kryochirurgiegeräten, Endoskopen oder dergleichen wird eine sichere Datenübertragung sichergestellt.

Es wird eine differentielle Übertragungstechnik gewählt, bei dem ein oder mehrere Leitungspaare (Adern 22, 23) genutzt werden, von denen jeweils einer der Leiter das Signal und der andere das invertierte Signal überträgt. Durch Subtraktion der beiden Signale wird empfängerseitig das eigentliche Datensignal gewonnen. Koppeln Störungen aus der Umgebung auf die Datenübertragungsstrecke, d.h. die Leitung ein, geschieht dies wegen der räumlichen Nähe und gegebenenfalls Verdrillung der beiden Leitungen im gleichen Maße. Durch die an den jeweiligen Empfängern vorgenommene Subtraktion heben sich die Störungen im Signal auf.

### Bezugszeichenliste:

- 10: chirurgisches System
- 11: Chirurgiegerät
- 12: chirurgisches Instrument
- 13: Leitung
- 14: Stecker
- 15: Buchse
- 16: Handgriff
- 17: Funktionselement, Werkzeug, Elektrode
- 18: Kupplung
- 19: Ader
- 20: Isolierstoff
- 21-23: Adern
- 24: Mikrocontroller
- 25: Bedienungselemente
- 26: Erster Taster
- 27: zweiter Taster
- 28: Potentiometer
- 29: Lagesensor
- 30: Identifizierungseinrichtung
- 31: Displayeinrichtung
- 32: Vibrationsgeber
- 33: Generator
- 34: Nullpotential
- 35: Neutralbuchse
- 36: Leitung
- 37: Leitung
- 38: Mikrocontroller
- 39: Gerätesteuerung
- 40: Potentialtrenneinrichtung
- 41: Versorgungseinheit

## Patentansprüche

1. Chirurgisches Instrument (12) zum Anschluss an ein speisendes Chirurgiegerät (11),
mit einem Handgriff (16), der über eine Leitung (13) mit einem Gerätestecker (14) zur Verbindung mit dem Chirurgiegerät (11) verbunden ist,
mit mindestens einem Funktionselement (17), das von dem Handgriff (16) gehalten und über die Leitung (13) von dem speisenden Chirurgiegerät (11) mit Wirkmedium zu versorgen ist,
mit mehreren Bedienelementen (25), die an dem Handgriff (16) angeordnet sind,
mit einem Mikrocontroller (24) der in dem Handgriff (16) angeordnet und mit den Bedienelementen (25) verbunden ist und der über die Leitung (13) mit elektrischer Energie versorgt ist und über Datenkommunikation mit dem Chirurgiegerät (11) Daten übertragend kommuniziert,
**dadurch gekennzeichnet, dass** in der Leitung (13) zwei Adern (22, 23) zur Datenübertragung vorgesehen sind, wobei der Mikrocontroller (24) an einer Ader (22) die zu übertragenden Daten und an der anderen Ader (23) die Daten in physikalisch invertiertem Zustand sendet und/ oder empfängt.

2. Instrument nach Anspruch 1, **dadurch gekennzeichnet, dass** an dem Handgriff (16) eine Anzeigeeinrichtung (31) vorgesehen sind, die mit dem Mikrocontroller (24) verbunden und von diesem gesteuert sind.

3. Instrument nach Anspruch 1, **dadurch gekennzeichnet, dass** die Anzeigeeinrichtung (31) sensorische, optische und/oder akustische Anzeigemittel sind.

4. Instrument nach Anspruch 1, **dadurch gekennzeichnet, dass** das Wirkmedium hochfrequenter elektrischer Strom ist, wobei die Leitung (13) eine HF-Spannung führende Ader (19) aufweist.

5. Instrument nach Anspruch 1, **dadurch gekennzeichnet, dass** das Wirkmedium ein Fluid oder Licht ist, wobei die Leitung (13) eine das Wirkmedium führende Ader (19) aufweist.

6. Instrument nach Anspruch 1, **dadurch gekennzeichnet, dass** in der Leitung (13) mindestens eine Ader (21) zur Spannungsversorgung des Mikrocontrollers (24) vorgesehen ist.

7. Instrument nach Anspruch 1, **dadurch gekennzeichnet, dass** in der Leitung (13) mindestens eine Ader (22) zur Datenübertragung vorgesehen ist, an die der Mikrocontroller (24) angeschlossen ist.

8. Instrument nach Anspruch 4 oder 5, **dadurch gekennzeichnet, dass** ein zweiter Mikrocontroller der im Chirurgiegerät 11 angeordnet ist an die HF-führende oder hochspannungsführende Ader (19) als Masse-Bezugspotential angeschlossen ist.

9. Instrument nach Anspruch 1, **dadurch gekennzeichnet, dass** die Daten auf der Leitung (13) als serieller Datenstrom übertragen werden.

10. Instrument nach Anspruch 1, **dadurch gekennzeichnet, dass** die Daten als Stromimpulse übertragen werden.

11. Instrument nach Anspruch 1, **dadurch gekennzeichnet, dass** die Mikrocontroller (24) zumindest einmal nach Anschluss an das Chirurgiegerät (11) einen instrumentenspezifischen Identifikationscode sendet.

12. Instrument nach Anspruch 1, **dadurch gekennzeichnet, dass** der Mikrocontroller (24) Signale der Bedienungselemente (25) in Befehle für das Chirurgiegerät (11) umsetzt.

13. Instrument nach Anspruch 3, **dadurch gekennzeichnet, dass** der Mikrocontroller (24) von dem Chirurgiegerät (11) über die Leitung (13) erhaltene Informationen mit der Anzeigeeinrichtung (31) darstellt.

14. Instrument nach Anspruch 1, **dadurch gekennzeichnet, dass** dem Mikrocontroller (24) ein in dem Chirurgiegerät (11) angeordneter zweiter Mikrocontroller (38) zugeordnet ist, der mit dem in dem Handgriff (16) untergebrachten Mikrocontroller (24) über die Leitung (13) elektrisch verbunden ist.

## Claims

1. Surgical instrument (12) for connection to a supplying surgery device (11),
with a handle (16) which is connected to a device plug (14) via a line (13) for connection to the surgery device (11),
with at least one functional element (17) which is held by the handle (16) and is supplied with active medium from the supplying surgery device (11) via the line (13), with several operating elements (25) which are arranged on the handle (16),
with a microcontroller (24) which is arranged in the handle (16) and connected to the operating elements (25), is supplied with electrical energy via the line (13) and communicates data-transmissively with the supplying surgery device (11) via data communication,
**characterised in that** two cores (22, 23) are provided in the line (13) for data transmission, wherein the microcontroller (24) transmits and/or receives the data to be transmitted on one core (22) and the data in physically inverted state on the other core (23).

2. Instrument according to claim 1, **characterised in that** a display device (31) is provided on the handle (16) and is connected to and controlled by the microcontroller (24).

3. Instrument according to claim 1, **characterised in that** the display device (31) comprises sensory, optical and/or acoustic display means.

4. Instrument according to claim 1, **characterised in that** the active medium is highfrequency electrical current, wherein the line (13) has a core (19) conducting HF voltage.

5. Instrument according to claim 1, **characterised in that** the active medium is a fluid or light, wherein the line (13) has a core (19) conducting the active medium.

6. Instrument according to claim 1, **characterised in that** in the line (13), at least one core (21) is provided for supplying voltage to the microcontroller (24).

7. Instrument according to claim 1, **characterised in that** in the line (13), at least one core (22) connected to the microcontroller (24) is provided for data transmission.

8. Instrument according to claim 4 or 5, **characterised in that** a second microcontroller, connected to the HF-conducting or high-voltage-conducting core (19) as a ground reference potential, is arranged in the surgery device (11).

9. Instrument according to claim 1, **characterised in that** the data are transmitted on the line (13) as a serial data stream.

10. Instrument according to claim 1, **characterised in that** the data are transmitted as current pulses.

11. Instrument according to claim 1, **characterised in that** the microcontroller (24) sends an instrument-specific identification code to the surgery device (11) at least once after connection.

12. Instrument according to claim 1, **characterised in that** the microcontroller (24) converts signals from the operating elements (25) into commands for the surgery device (11).

13. Instrument according to claim 3, **characterised in that** the microcontroller (24) displays information received from the surgery device (11) via the line (13) by means of the display device (31).

14. Instrument according to claim 1, **characterised in that** a second microcontroller (38) arranged in the surgery device (11) is assigned to the microcontroller (24) and is electrically connected via the line (13) to the microcontroller (24) accommodated in the handle (16).

## Revendications

1. Instrument chirurgical (12) destiné à être raccordé à un appareil chirurgical (11) d'alimentation,
comprenant un manche (16) qui est relié par un câble (13) à un connecteur d'appareil (14), en vue du raccordement à l'appareil chirurgical (11),
comprenant au moins un élément fonctionnel (17) qui est tenu par le manche (16) et est alimenté avec un agent actif par l'appareil chirurgical (11) d'alimentation via le câble (13),
comprenant plusieurs éléments de commande (25) qui sont disposés sur le manche (16),
comprenant un microcontrôleur (24) qui est disposé dans le manche (16) et est relié aux éléments fonctionnels (25) et qui est alimenté en énergie électrique par l'intermédiaire du câble (13) et communique avec l'appareil chirurgical (11) en transférant des données par transmission de données,
**caractérisé en ce qu'**il est prévu dans le câble (13) deux fils (22, 23) destinés à la transmission de données, le microcontrôleur (24) émettant les données à transmettre sur un fil (22) et/ou recevant sur l'autre fil (23) les données à l'état physiquement inversé.

2. Instrument selon la revendication 1, **caractérisé en ce qu'**il est prévu sur le manche (16) un dispositif d'affichage (31) qui est relié au microcontrôleur (24) et est commandé par celui-ci.

3. Instrument selon la revendication 1, **caractérisé en ce que** le dispositif d'affichage (31) est constitué de moyens d'affichage optiques, acoustiques et/ou à base de capteurs.

4. Instrument selon la revendication 1, **caractérisé en ce que** l'agent actif est un courant électrique à haute fréquence, le câble (13) présentant un fil (19) qui est parcouru par une tension HF.

5. Instrument selon la revendication 1, **caractérisé en ce que** l'agent actif est un fluide ou de la lumière, et dans ce cas le câble (13) présente une conduite (19) parcourue par l'agent actif.

6. Instrument selon la revendication 1, **caractérisé en ce qu'**il est prévu dans le câble (13) au moins un fil (21) qui est destiné à l'alimentation en tension du microcontrôleur (24).

7. Instrument selon la revendication 1, **caractérisé en ce qu'**il est prévu dans le câble (13) au moins un fil (22) qui est destiné à la transmission de données et auquel est raccordé le microcontrôleur (24).

8. Instrument selon la revendication 4 ou 5, **caractérisé en ce qu'**un deuxième microcontrôleur, disposé dans l'appareil chirurgical (11), est raccordé en tant que potentiel de référence de masse au fil (19) parcouru par le courant HF ou par la haute tension.

9. Instrument selon la revendication 1, **caractérisé en ce que** les données sont transmises sur le câble (13) sous la forme d'un flux de données en série.

10. Instrument selon la revendication 1, **caractérisé en ce que** les données sont transmises sous forme d'impulsions de courant.

11. Instrument selon la revendication 1, **caractérisé en ce que** le microcontrôleur (24) émet un code d'identification spécifique à l'instrument, au moins une fois après le raccordement à l'appareil chirurgical (11).

12. Instrument selon la revendication 1, **caractérisé en ce que** le microcontrôleur (24) convertit des signaux des éléments de commande (25) en instructions pour l'appareil chirurgical (11).

13. Instrument selon la revendication 3, **caractérisé en ce que** le microcontrôleur (24) représente via le dispositif d'affichage (31) les informations reçues de l'appareil chirurgical (11) par l'intermédiaire du câble (13).

14. Instrument selon la revendication 1, **caractérisé en ce qu'**un deuxième microcontrôleur (38), placé dans l'appareil chirurgical (11), est associé au microcontrôleur (24) et est relié électriquement via le câble (13) au microcontrôleur (24) disposé dans le manche (16).
